# EUROPEAN PATENT APPLICATION

(11) **EP 1 340 817 A1**
(43) Date of publication of application: **03.09.2003**
(21) Application number: 03251012.5
(22) Date of filing: 19.02.2003
(51) Int. Cl.: C12Q 1/48, C12N 9/12

(54) **Assay for activity of the actRIIB kinase**

(30) Priority: 28.02.2002 US 360607 P
(71) Applicant: Pfizer Products Inc., Groton, Connecticut 06340 (US)
(72) Inventor: Krasney, Philip A., c/o Pfizer Global Res. & Dev., Groton, Connecticut 06340 (US); Norcia, Michael, c/o Pfizer Global Res. & Dev., Groton, Connecticut 06340 (US); O'Connor, Barbara A, c/o Pfizer Global Res. & Dev., Groton, Connecticut 06340 (US); Spencer, Robin W., c/o Pfizer Global Res. & Dev., Groton, Connecticut 06340 (US)
(74) Representative: England, Peter Michael

(57) **Abstract**

The present invention relates to kinase assays, and specifically to novel kinase assay methods using a novel target peptide for measuring the activity and/or modulation of activity of the ActRIIB kinase protein.

## Description

### FIELD OF THE INVENTION

The present invention relates to kinase assays, and specifically to novel kinase assay methods for measuring the activity and/or modulation of the activity of ActRIIB kinase.

### BACKGROUND OF THE INVENTION

Activins were originally discovered as gonadal polypeptide hormones that stimulate follicle-stimulating hormone (FSH) secretion in cultured pituitary cells. There are three activins (A, B, and AB) that are homo/heterodimers of two closely related *β subunits (β*_{*A*}*β*_{*A*}*,* β_{B}β_{B}, *and* β_{A}β_{B}) (Vale et al. in *Peptide Growth Factors and Their Receptors: Handbook of Experimental Pharmacology,* vol. 95, p. 221, Sporn and Roberts (Eds.), Springer-Verlag (New York, NY, 1990)). Erythroid differentiation factor (EDF) was first found in the culture fluid of phorbol myristate acetate (PMA)-treated human monocytic leukemic cells (THP-1) based on its ability to induce erythroid differentiation of murine Friend erythroleukemia cells (Eto et al., *Biochem. Biophys. Res. Comm.,* 142:1095 (1987)). Later, it turned out to be identical to activin A, because it was encoded by the same mRNA as the β_{A} subunit of activin A (Murata et al., *PNAS,* 85:2434 (1988)). Activin A subsequently was shown to induce hemoglobin synthesis in human K562 erythroleukemia cells (Yu et al., *Nature,* 330:765 (1987)). Moreover, it enhances the growth of normal erythroid precursor cells both in vitro (Yu, supra; Broxmeyer, *PNAS,* 85:9052 (1988)) and in vivo (Shiozaki et al., *Biochem. Biophys. Res. Comm.,* 165:1155 (1989); Schwall et al., *Endocrinology,* 125:1420 (1989)). Activin A and its mRNA are expressed in the bone marrow and spleen of adult rodents (Meunier et al., *PNAS,* 85:247 (1988); Shiozaki et al., *PNAS,* 89:1553 (1992)). Activin A is also produced by murine and human bone marrow stromal cells in culture (Yamashita et al., *Blood,* 79:304 (1992); Shao et al., *Exp. Hematol.,* 20:1235 (1992)). Furthermore, it was recently shown to be secreted by human peripheral blood monocytes (Shao, supra; Erämaa et al., *J*. *Exp. Med.,* 176:1449 (1992)). These findings suggest that activin A acts as a natural regulator of erythropoiesis in the bone marrow.

The biologic effects of activin are expected to be mediated through type I and type II receptors (Massague, *Cell,* 69:1067 (1992)). Presently, there are known
to be two type I receptors, ActRl and ActRIA, as well as two homologous type II activin receptors, ActRll and ActRIIB, all of which have been characterized by cDNA cloning. ActRll and ActRIIB are transmembrane proteins with an intracellular kinase domain structurally related to several known serine/threonine kinases (Mathews et al., *Cell,* 65:973 (1991); Attisano et al., *Cell,* 68:97-108 (1992)).

Other references about activin receptors include Mathews et al., *Science,* 255:1702-05 (1992); Mathews et al., *J*. *Biol. Chem.,* 268:19013-18 (1993); and Hilden et al., *Blood,* 83:2163-70 (1994).

ActRIIB is a single-transmembrane receptor kinase. Myostatin (a member of the TGF-beta (Transforming Growth Factor) superfamily, also called Growth and Differentiation Factor 8 or GDF-8) is one of ActRIIB kinase's natural ligands in skeletal muscle. Signaling by myostatin through ActRIIB kinase results in the negative regulation of skeletal muscle mass. Thus, inhibitors of the ActRIIB kinase activity may prevent or reverse the age-related loss of muscle mass that contributes to frailty; and it is thus a goal of the present invention to provide a useful assay for screening, especially high throughput screening, of potential ActRllB kinase ligands, as the discovery of such ligands may lead to the discovery of suitable drug therapies for frailty.

Protein kinases are enzymes which covalently modify proteins and peptides by the attachment of a phosphate group to one or more sites on the protein or peptide. Protein kinases represent one of the largest group of enzymes, with critical role in many cellular signal transduction processes. Due to genome projects and other recent developments in molecular biology techniques, new kinases with uncharacterized biochemical properties and substrate specificity are discovered more and more frequently. Traditional protein kinase assays include the use of labeled ATP as phosphodonor, and a substrate peptide as phosphoacceptor containing the respective kinase recognition motif. Following the kinase reaction the substrate peptide is captured on an appropriate filter. Unreacted labeled ATP and metabolites are resolved from the radioactive peptide substrate by various techniques, involving trichloroacetic acid precipitation and extensive washing. Addition of several positively charged residues allows capture on phosphocellulose paper followed by washing. Radioactivity incorporated into the substrate peptide is detected by scintillation counting. This assay is relatively simple, reasonably sensitive, and the peptide substrate can be adjusted both in terms of sequence and concentration to meet the assay requirements. See U.S. Patent 5,759,787 for more details on exemplary kinase assays.

### SUMMARY OF THE INVENTION

In a first aspect, the present invention provides an assay for determining the kinase activity of ActRllB, said assay comprising the steps of incubating ActRIIB, labelled phosphodonor, and a target peptide in an suitable medium such that said ActRIIB may phosphorylate said target peptide using said labelled phosphodonor, thereby creating a labelled target peptide, and measuring the amount of label present in said labelled target peptide.

In a second aspect, the present invention provides An assay for discovering modulators of ActRIIB kinase, said assay comprising the steps of incubating in a first reaction ActRIIB kinase, labelled phosphodonor, and a target peptide in an appropriate medium such that said ActRIIB kinase may phosphorylate said target peptide using said labelled phosphodonor, thereby creating a labelled target peptide, and incubating in a second reaction ActRllB kinase, labelled phosphodonor, target peptide, and a test compound, measuring the amount of label present in said labelled target peptide from said first and second reactions, and comparing the amount of labelled target peptide in said second reaction to the amount of labelled target peptide from said first reaction, wherein any statistically significant difference in the amount of labelled target peptide indicates that said test compound is a modulator of ActRIIB kinase.

In a third aspect, the present invention provides the protein catActRllB kinase, wherein said protein has the sequence shown in SEQ ID NO:2.

### DESCRIPTION OF THE DRAWING

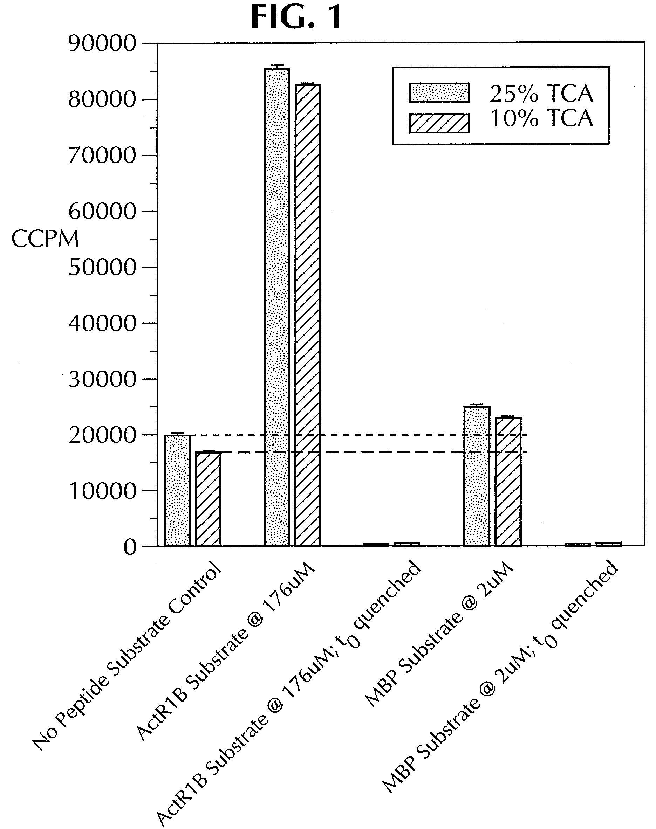

Figure 1 provides a graphical comparison of the results of several kinase reactions run using the scintillation counting method.

### DETAILED DESCRIPTION

Those skilled in the art will fully understand the terms used herein in the description and the appendant claims to describe the present invention. Nonetheless, unless otherwise provided herein, the following terms are as described immediately below.

By "ActRllB" is meant the protein Activin Receptor IIB, or sometimes the gene encoding the Activin Receptor IIB protein. "ActRllB kinase" is equivalent to simply "ActRIIB", though it is typically used when it is desirable to emphasize the kinase activity and/or the kinase catalytic portion of ActRllB. Unless specified, ActRIIB refers to the protein as found in human, mouse, or any other organism (See, for example, Hilden et al., *Blood,* 83(8):2163-70 (1994) for human; Attisano et al., *Cell,* 68:97-108 (1992) for mouse; and Mathews et al., *Science,* 255:1702-05 (1992) for Xenopus). Additionally, unless specified, ActRIIB refers to all naturally occurring alleles of the protein, as well as man-made (e.g., genetically engineered) variants thereof.

By "catActRllB" is meant a modified version of the ActRIIB protein designed for use in the present invention. ActRIIB is modified by retaining the catalytic portion and removing hydrophobic portions that are unnecessary for enzymatic activity, thus creating an enzyme with superior solubility characteristics. While unmodified ActRllB will work in the inventions methods, this modified version is presently preferred. catActRllB preferably consists of amino acids 167-512 of human ActRIIB (SEQ ID NO:2). These amino acids correspond to the catalytic (cytosolic) domain of the protein, and also correspond to nucleotides 503-1543 of accession #77533 (see Hilden at al., *Blood,* 83(8):2163-70 (1994)).

By "his-ActRllB" "his-catActRllB" is meant the ActRIIB or catActRllB protein expressed from a construct that has been genetically engineered such that the protein includes a histidine or his6 tag for additional ease of purification.

By "phosphodonor" is meant a compound that is involved in the enzymatic reaction catalyzed by ActRIIB, wherein the phosphodonor loses a phosphoryl group that is transferred to the substrate by ActRllB. The most common phosphodonors are nucleotide triphosphates and diphosphates. The presently most preferred phosphodonor for use in the present invention is ATP.

By "reduced" is meant a statistically significant decrease (i.e., p<0.1).

By "increased" is meant a statistically significant increase (i.e., p<0.1).

By "modulates" is meant a statistically significant increase or decrease (including a complete elimination).

In its most basic form, a kinase assay is simply a combination of a kinase protein, an appropriate substrate, a phosphate donor, and a means for measuring the amount of reaction that occurs. Of course, within this simplistic plan can lie a multitude of complications. In the case of the present invention, a problem occurred when it was attempted to create an assay for measuring the kinase activity of ActRllB, or more specifically, a modified version of ActRllB (referred to herein as catActRllB, see hereinbelow). A commonly used substrate for kinase assays is Myelin Basic Protein, or MBP. It is generally a good target for the majority of kinases, and is easily and cheaply obtained. However, when it was attempted to use MBP as a substrate for measuring the kinase activity of catActRllB, the attempt surprisingly failed (see Examples 1 and 2 herein). Thus, it was necessary for another substrate to be used.

The substrate for the kinase assay of the present invention is referred to as the "target peptide". This small peptide is an excellent substrate for the ActRllB protein, in all its forms as described herein. It contains the necessary recognition motif for specific recognition by ActRllB, as well as the necessary amino acids to be phosphorylated. In a most preferred embodiment, the target peptide is 27 amino acids in length, having the sequence: H₂N-Val-Tyr-Asp-Leu-Ser-Thr-Ser-Gly-Ser-Gly-Ser-Gly-Leu-Pro-Leu-Phe-Val-Gln-Arg-Thr-Val-Ala-Arg-Thr-lle-Val-Leu-COOH (SEQ ID NO:1). It is believed that ActRIIB recognizes the amino acid sequences STSGSGS and RTVART when they are appropriately spaced, as in the preferred target peptide. However, alternative peptide sequences are also expected to be functional in the present assay, as long as they sufficiently resemble SEQ ID NO:1 so as to be effectively recognized by ActRllB.

In order for a kinase assay to be useful, it must be possible to measure the reaction, i.e., to determine how much phosphorylation has been catalyzed by the kinase in a known amount of time. The simplest presently known method of doing this is to provide one reactant of the phosphorylation reaction that provides a label which can be measured in the product (and the labelled product differentiated from the labelled reactant). Most commonly, this is done by providing radioactively labelled ATP, the use of which in the kinase reaction will result in radioactively labelled target peptide. Since the phosphoryl group transferred from ATP to the target peptide contains atoms of oxygen and phosphorous, it is theoretically possible to use radioactive isotopes of any of these atoms as the label. In practice, phosphorous (³²P or ³³P) is the preferred choice. In addition to direct radioactive labeling, there are indirect labeling or capture methods that exploit antibodies specific for the phosphorylated form of the peptide. This approach forms the basis of both radio-immunoassays and non-radiometric immunoassays. In the latter, the phosphopeptide-specific antibody (or secondary antibody) may carry an integral enzyme actvity, such as horseradish peroxidase, which will allow detection by use of a chromogenic substrate, or, the antibody may carry some easily detected fluorophore or phosphor. If the substrate peptide includes an appropriate fluorophor that does not interfere with its suitability as a substrate, the phospopeptide specific antibody can be employed in a fluorescence polarization detection scenario. Since the non-phosphorylated peptide will have more rapid rotational diffusion compared to phosphorylated peptide/antibody complex, these two forms of the substrate (unbound and antibody-bound) are distinguishable upon analysis using polarized light. In a presently preferred embodiment, ATP containing ³²P or ³³P is used, wherein the total concentration of ATP in the reaction mixture is between about 7.4 µM - 7.6 *µM,* with approximately 0.001% - 0.004% of the total ATP being radioactively labelled. This results in preferred radioactivity levels of about 0.2 - 0.5 µCi/ml of reaction volume.

As is understood by those skilled in the art, in addition to the kinase enzyme itself and the necessary reactants, for effective and repeatable assay measurements it is also necessary that the reaction occur in an appropriate media composed of appropriate solvent(s), salts, and various factors that facilitate the reaction. Water is the preferred media, but other solvents may be used in whole or more preferably in combination with water. These include DMSO, ethanol, and other solvents known to those of skill in the art as being potentially compatible with enzymatic activity. Of course, it is preferred that buffers be included in the assay to maintain an appropriate pH range. Useful buffers include HEPES, Tris, MOPS, and the like. The pH is preferably about 6.3 - 8.3, and more preferably about 6.8 - 8.8. In a most preferred embodiment, the pH is about 7.3. It is important to include certain salts in the reaction mixture for optimal activity. In particular, it is preferred to include MgCl₂ and MnCl₂ at appropriate concentrations. Finally, certain surfactants, cofactors, and the like are preferably included. Among these are BSA or other stability-enhancing proteins, as well as EDTA or other heavy metal scavenging compounds.

It is preferable to incubate the kinase reaction of the present assay at a temperature between 50-100°F. More preferably, the reaction occurs at between 65-80°F. Because it is preferred and easily achieved, the reaction is best incubated at room temperature (typically about 72°F). This incubation period can last anywhere from a few minutes to a few hours, with a time period of 20 minutes to 90 minutes being preferred. More preferred is an incubation time of between 40 and 80 minutes, with 75 minutes being presently most preferred.

After running the kinase assay, it is necessary to stop the ActRIIB enzyme reaction at a pre-determined time. If the reaction isn't stopped at a precise known time, then it isn't feasible to compare results from one reaction to another. Of course, any reasonable method of stopping the reaction may be utilized, as long as it doesn't interfere with accurate measurement of the kinase reaction results. For example, certain compounds may be added to the reaction mixture that rapidly denature, degrade, or otherwise disable the ActRIIB protein. Such compounds include TCA, phosphoric acid, SDS, and the like. Alternatively, it may be feasible to heat the reaction mixture to the point where the ActRIIB protein is permanently denatured. Such action would require heating to a temperature of at least about 160°F. Other suitable means are known to those skilled in the art.

After the kinase reaction is complete, and the ActRIIB protein activity is disabled, isolation of the labelled target substrate is typically required. If the target substrate is not isolated, it is typically impossible to distinguish the signal generated by the labelled substrate from the signal generated by unused labelled reactant. In some cases, however, it is not necessary to isolate the labelled target substrate, and in such cases immediate measurement is performed. For example, in certain cases an assay such as a scintillation proximity assay may be performed, in which case isolation of the labelled substrate is unnecessary.

Those of skill in the art are aware of many means for isolating the labelled target substrate from the unused labelled reactant. Typical examples include gel electrophoresis, precipitation, filtration, chromatography, immunoprecipitation, and the like. Presently, it is preferred to separate the labelled target substrate by TCA precipitation of the target peptide.

An excellent method of separation is specific binding of the labelled target substrate to a solid support followed by washing away of the unused labelled reactant. For this method, a wide variety of solid substrates may be used. Factors to be considered in selecting an appropriate substrate include the adhesion and functional retention of the immobilizing receptor, accessible surface area for binding, wash convenience, cost, high-throughput adaptability, etc. Frequently, the solid substrate will be the wall of the reaction reservoir itself. Preferred substrates maximize signal strength and the signal-to-noise ratio. Exemplary substrates include polystyrene microtiter plates, fine fibers, polymeric or silica-based microbeads, etc., preferably pre-activated to provide maximal protein binding. When used, microbeads are selected by size, range and structure to maximize surface area, filter retention and bead suspension time during the assay incubations.

Once the labelled target substrate is separated from unused labelled reactant, it is necessary to measure the amount of labelled target substrate. The means of making this measurement depend upon the type of label used. For radioactively labelled target substrates, the amount of radioactivity present is measured in any of a variety of means known to those of skill in the art, including scintillation counting, quantitative autoradiography, densitometry, phosphoimaging, and the like.

If the target substrate is labelled with a fluorescent label, then the amount of label may be measured by quantitative spectrophotometry, fiuorescence/chemiluminescence imaging, or the like.

Other methods for detecting kinase activity are based on separations due to the charge differences between phosphorylated and non-phosphorylated proteins and peptides. In these respects, techniques based on gel electrophoresis and HPLC have, among others, been used. In combination with these techniques, spectrophotometric and fluorometric detection have been used. Reference is made to International Patent Application WO 93/10461 and U.S. Pat. Nos. 5,120,644 and 5,141,852 for descriptions of many methods heretofore used for detecting protein kinase activity. Also reference is made to Toomik et al., *Analytical Biochemistry,* 209:348-53 (1993).

In addition to the kinase, substrate, NTP, and first receptor, the reaction mixture may also comprise a test compound such as a preselected kinase inhibitor or modulator or, especially for high-throughput drug screening, a library-derived test compound. Quite simply, through measurement of the kinase reaction, it is readily possible to determine if the kinase reaction is increased or decreased through the presence of the test compound. If the reaction rate is increased, the test compound is a likely agonist. If the reaction rate is decreased, the test compound is a likely antagonist.

Library-derived test compounds encompass numerous chemical classes, though typically they are organic compounds; preferably small organic compounds. The libraries may comprise synthetic and/or naturally derived compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural and synthetically produced libraries and compounds are readily modified through conventional chemical, physical, and biochemical means. In addition, known pharmacological agents may be subject to directed or random chemical modifications, such as acylation, alkylation, esterification, amidification, etc., to produce structural analogs. The agent is provided in standard serial dilutions or in an amount determined by analogy to known modulators. In addition, the mixture usually includes additional reagents, such as salts, buffers, etc., to facilitate or maximize kinase activity.

While not strictly necessary, as a practical matter it is highly useful to include a series of controls for the kinase reactions. If a test compound is being added to one reaction mixture, it is important to add an identical volume of a similar composition (absent the test compound only) to a control kinase reaction. This will then account for any alterations in kinase activity caused by solvents, salts, or other components of the solution containing the test compound.

It is also very helpful to include positive and negative control kinase reactions that are likewise as similar as possible to the experimental reactions, but which contain known modulators of the kinase activity. By testing known inhibitors and agonists of the kinase activity alongside the unknown test compounds, it is easier to control for unforeseeable fluctuations in kinase responsiveness.

One of the most important aspects of the present invention is its suitability for use in high throughput screening (HTS) for modulators of ActRllB kinase. Because the reaction is simple, performable in small volumes (e.g., on microtiter plates), and reproducible, it is possible to screen huge libraries of compounds and discover those that modulate the activity of ActRIIB kinase in favorable manners. Such newly discovered modulators are potential pharmaceuticals for humans and animals.

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art. Additional guidance is found in standard textbooks of molecular biology, protein science, immunology, and the like. All publications cited in this document are herein incorporated by reference in their entirety.

### EXAMPLES

### Example 1 - Autoradiographic Assay Method

A 20 µl reaction mixture comprising 2.5 µg/ml his-catActRllB kinase, 1 mg/ml target peptide or 2.5 µM myelin basic protein (MBP), 10 µM ATP, 10 µCi [³²P] y-ATP (6000 Ci/mmol), 1 mg/ml bovine serum albumin (BSA), and 5 mM sodium orthovanadate in 25 mM HEPES (pH 7.3) was run for 30 minutes at ambient temperature. The reaction was stopped by adding 1/3 volume of 3x Laemmli buffer with β-mercaptoethanol and trace bromphenol blue (188 mM Tris pH 6.8, 3% SDS, 24% glycerol, 25% v/v β-mercaptoethanol). The stopped reaction was loaded directly onto a 18% ployacrylamide/tris/glycine gel without boiling. The gel was run at about 100 volts to permit proper separation, washed for five minutes in water, and equilibrated for five minutes in semi-dry blotting buffer (48 mM Tris base, 39 mM glycine, 20% methanol, 0.375% SDS). Reaction products were then blotted onto a nitrocellulose filter for 48 minutes at 20 volts in a Bio-Rad semi-dry blotting apparatus and visualized and/or quantitated by autoradiography.

In the reaction mixture comprising target peptide, bands were visualized at 3 Kd and 40 Kd, corresponding to labelled target peptide and autophosphorylated his-catActRIIB kinase, respectively. In the reaction mixture comprising MBP, bands were visualized at 18 Kd and 40 Kd, corresponding to labelled MBP and autophosphorylated his-catActRllB kinase, respectively. Finally, in a control reaction mixture that contained neither target peptide nor MBP, the only band visualized was the autophosphorylated his-catActRllB kinase at 40 Kd. It is therefore clear that the present reaction conditions are conducive to ActRIIB-catalyzed phosphorylation (kinase) activity.

### Example 2 - Scintillation Method

A series of reaction mixtures comprising 25 mM HEPES (pH 7.3), 3 mM MgCl₂, 1 mM MnCl₂, 0.1 mM EDTA, 1 mM DTT, 0.2 mM sodium orthovanadate, 100 µg/ml BSA, 10 µM ATP, 0.25 µCi [³³P] y-ATP, and 16.5 µg/ml his-catActRllB kinase was run in a total of 100 µl directly in a Durapore (Millipore) filter plate for 80 min at room temperature. The reaction was stopped by addition of an equal volume of ice cold TCA (10% or 25% v/v). After one hour at 4°C, the precipitate was collected under vacuum. The precipitate was washed five times with cold TCA. After drying overnight, scintillant was added to the well and plates counted in a Microbeta 1450 Trilux in the Paralux mode. Different reactions included either the target peptide (176 µM), MBP (2 µM), or no substrate. The results are shown in Figure 1.

As seen in these results, even though it is known from the autoradiographic method that his-catActRllB phosphorylates the MBP, the reaction with MBP unexpectedly yields a negative result. The only signal generated is attributable to autophosphorylation of the his-catActRllB protein, as seen in the negative control (no substrate). However, the reaction performed very well with the target peptide as a substrate.

## Claims

1. An assay for determining the kinase activity of ActRllB, said assay comprising the steps of:
a) incubating ActRllB, labelled phosphodonor, and a target peptide in an suitable medium such that said ActRllB may phosphorylate said target peptide using said labelled phosphodonor, thereby creating a labelled target peptide; and
b) measuring the amount of label present in said labelled target peptide.

2. The assay of claim 1, wherein said target peptide has the sequence shown in SEQ ID NO:1.

3. The assay of claim 1, wherein said ActRllB is catActRllB.

4. The assay of claim 1, further comprising the step of isolating said labelled target peptide from any remaining labelled phosphodonor prior to said measuring step.

5. The assay of claim 1, wherein said incubating step further comprises the addition of a potential inhibitor of ActRllB.

6. An assay for discovering modulators of ActRIIB kinase, said assay comprising the steps of:
a) incubating in a first reaction ActRllB kinase, labelled phosphodonor, and a target peptide in an appropriate medium such that said ActRllB kinase may phosphorylate said target peptide using said labelled phosphodonor, thereby creating a labelled target peptide, and incubating in a second reaction ActRllB kinase, labelled phosphodonor, target peptide, and a test compound;
b) measuring the amount of label present in said labelled target peptide from said first and second reactions; and
c) comparing the amount of labelled target peptide in said second reaction to the amount of labelled target peptide from said first reaction, wherein any statistically significant difference in the amount of labelled target peptide indicates that said test compound is a modulator of ActRIIB kinase.

7. The assay of claim 6, wherein said target peptide has the sequence shown in SEQ ID NO:1.

8. The assay of claim 6, wherein said ActRIIB is catActRllB.

9. The assay of claim 6, further comprising the step of isolating said labelled target peptide from any remaining labelled phosphodonor in said first and second reactions prior to said measuring step.

10. The protein catActRllB kinase, wherein said protein has the sequence shown in SEQ ID NO:2.


### SEQUENCE LISTING

<110>  PFIZER PRODUCTS INC.

<120>  ASSAY FOR ACTIVITY OF THE ActRIIB KINASE

<130>  PC11691A

<140>  60/360,607
<141>  2002-02-28

<160>  2

<170>  PatentIn version 3.1

<210>  1
<211>  27
<212>  PRT
<213>  Artificial

<220>
<223>  Artifical sequence

<400>  1

Val Tyr Asp Leu Ser Thr Ser Gly Ser Gly Ser Gly Leu Pro Leu Phe
1               5                   10                  15

Val Gln Arg Thr Val Ala Arg Thr Ile Val Leu
            20                  25

<210>  2
<211>  346
<212>  PRT
<213>  Homo sapiens

<400>  2

Tyr Gly His Val Asp Ile His Glu Asp Pro Gly Pro Pro Pro Pro Ser
1               5                   10                  15

Pro Leu Val Gly Leu Lys Pro Leu Gln Leu Leu Glu Ile Lys Ala Arg
            20                  25                  30

Gly Arg Phe Gly Cys Val Trp Lys Ala Gln Leu Met Asn Asp Phe Val
        35                  40                  45

Ala Val Lys Ile Phe Pro Leu Gln Asp Lys Gln Ser Trp Gln Ser Glu
    50                  55                  60

Arg Glu Ile Phe Ser Thr Pro Gly Met Lys His Glu Asn Leu Leu Gln
65                  70                  75                  80

Phe Ile Ala Ala Glu Lys Arg Gly Ser Asn Leu Glu Val Glu Leu Trp
                85                  90                  95

Leu Ile Thr Ala Phe His Asp Lys Gly Ser Leu Thr Asp Tyr Leu Lys
            100                 105                 110

Gly Asn Ile Ile Thr Trp Asn Glu Leu Cys His Val Ala Glu Thr Met
        115                     120                     125

Ser Arg Gly Leu Ser Tyr Leu His Glu Asp Val Pro Trp Cys Arg Gly
    130                     135                     140

Glu Gly His Lys Pro Ser Ile Ala His Arg Asp Phe Lys Ser Lys Asn
145                     150                     155                     160

Val Leu Leu Lys Ser Asp Leu Thr Ala Val Leu Ala Asp Phe Gly Leu
                165                     170                     175

Ala Val Arg Phe Glu Pro Gly Lys Pro Pro Gly Asp Thr His Gly Gln
            180                     185                     190

Val Gly Thr Arg Arg Tyr Met Ala Pro Glu Val Leu Glu Gly Ala Ile
        195                     200                     205

Asn Phe Gln Arg Asp Ala Phe Leu Arg Ile Asp Met Tyr Ala Met Gly
    210                     215                     220

Leu Val Leu Trp Glu Leu Val Ser Arg Cys Lys Ala Ala Asp Gly Pro
225                     230                     235                     240

Val Asp Glu Tyr Met Leu Pro Phe Glu Glu Glu Ile Gly Gln His Pro
                245                     250                     255

Ser Leu Glu Glu Leu Gln Glu Val Val Val His Lys Lys Met Arg Pro
            260                     265                     270

Thr Ile Lys Asp His Trp Leu Lys His Pro Gly Leu Ala Gln Leu Cys
        275                     280                     285

Val Thr Ile Glu Glu Cys Trp Asp His Asp Ala Glu Ala Arg Leu Ser
    290                     295                     300

Ala Gly Cys Val Glu Glu Arg Val Ser Leu Ile Arg Arg Ser Val Asn
305                     310                     315                     320

Gly Thr Thr Ser Asp Cys Leu Val Ser Leu Val Thr Ser Val Thr Asn
                325                     330                     335

Val Asp Leu Pro Pro Lys Glu Ser Ser Ile
            340                     345